(19) Europäisches Patentamt / European Patent Office / Office européen des brevets

(11) **EP 3 077 005 B1**

(12) **EUROPEAN PATENT SPECIFICATION**

(45) Date of publication and mention
of the grant of the patent:
**04.08.2021 Bulletin 2021/31**

(51) Int Cl.:
*A61K 31/14* *(2006.01)*    *A61K 31/202* *(2006.01)*
*A61K 31/355* *(2006.01)*    *A61K 31/375* *(2006.01)*
*A61K 31/4415* *(2006.01)*    *A61K 31/519* *(2006.01)*
*A61K 31/7068* *(2006.01)*    *A61K 31/7072* *(2006.01)*
*A61K 31/714* *(2006.01)*    *A61K 33/04* *(2006.01)*
*A61K 45/06* *(2006.01)*    *A61P 25/02* *(2006.01)*
*A61P 25/14* *(2006.01)*    *A61P 25/16* *(2006.01)*
*A23L 33/10* *(2016.01)*    *A61P 1/10* *(2006.01)*

(21) Application number: **14815077.4**

(22) Date of filing: **30.09.2014**

(86) International application number:
**PCT/NL2014/050673**

(87) International publication number:
**WO 2015/084161 (11.06.2015 Gazette 2015/23)**

(54) **COMPOSITION COMPRISING A URIDINE SOURCE AND AN OMEGA-3 PUFA FOR IMPROVING COORDINATION, BALANCE, GRIP STRENGTH OR FINE MOTOR SKILLS**

ZUSAMMENSETZUNG MIT EINER URIDINQUELLE UND EINER OMEGA-3-PUFA ZUR VERBESSERUNG DER KOORDINATION, BALANCE, GRIFFKRAFT ODER FEINMOTORIK

COMPOSITION COMPRENANT UNE SOURCE D'URIDINE ET UN ACIDE GRAS POLYINSATURÉ D'OMÉGA 3 SERVANT À AMÉLIORER LA COORDINATION, L'ÉQUILIBRE, LA FORCE DE PRÉHENSION OU LA MOTRICITÉ FINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2013 PCT/NL2013/050879**

(43) Date of publication of application:
**12.10.2016 Bulletin 2016/41**

(60) Divisional application:
**21180324.2**

(73) Proprietor: **N.V. Nutricia**
**2712 HM Zoetermeer (NL)**

(72) Inventor: **ATTALI, Amos**
**NL-2712 HM Zoetermeer (NL)**

(74) Representative: **V.O.**
**P.O. Box 87930**
**Carnegieplein 5**
**2508 DH Den Haag (NL)**

(56) References cited:
**WO-A1-2009/002145    WO-A1-2009/002146**
**WO-A1-2012/125020**

• **None**

**Description**

The invention relates to a composition comprising a uridine source, an omega-3 polyunsaturated fatty acid for use in the improvement of a subject's coordination, balance, grip strength or fine motor skills.

The nervous system, in particular the brain, plays an essential role in a mammal's life. For example cognitive, emotional, social, sensory, motoric and regulatory functions are mainly determined by the brain. In society more and more diseases, disorders and problems with a proper functioning of the brain are becoming recognised. Brain functioning can become impaired, e.g. due to traumata (e.g. accidents, violence), abuse of drugs (e.g. alcohol) or systematic malnourishment. Also during aging several deleterious changes occur in the nervous system and in particular in the brain. Malfunctioning of the brain is therefore especially a problem in the elderly and more in particular in the frail or malnourished elderly. In this group, several diseases and disorders which are associated with or find their cause in a badly functioning nervous system are therefore relatively frequently observed. Examples are several forms of dementia, progressive neurodegenerative disorders, mood disorders but also other diseases or disorders like abnormal behaviour.

Several nutritional approaches have been proposed in the art to support brain function. For instance, WO 2009/002165 relates to a lipid fraction for the support of brain function. The lipid fraction comprises hexanoic acid and/or octanoic acid, eicosapentaenoic acid, and more than 0.4 g [alpha]-linolenic acid per 100 g fatty acids of the lipid fraction for the support of brain function. Optionally, the lipid fraction is administered in combination with non-lipid ingredients, but the lipid fraction preferably contributes to more than 45 en% of the energy of a ready to consume product. Nucleotides may be added to support brain function. Use of fibres is mentioned in order to reduce gastro-intestinal discomfort or to improve postprandial lipid profile. WO 2009/002165 acknowledges that brain function involves various distinct aspects, such as the cognition, social skills, decision making skills and motoric skills. However, even though motoric skills are mentioned as something in which brain function plays a role, there is no specific disclosure of the use of specific combination of components in order to provide a composition that is particularly useful for improving or maintaining the functioning of mammal's limbs.

WO 2009/002146 is directed to a composition comprising DHA and/or EPA plus uridine or its equivalent for use in supporting activities in daily living, such as independent housekeeping, transportation, shopping or personal hygiene activities, independent walking, bathing, grooming, dressing, use of communication equipment, use of household appliances, cleaning dishes, preparation of meal or drink or writing. The disclosed compositions lack the presence of a butyrate producing fibre.

WO 2012/125020 relates to a composition comprising a uridine or cytidine source , a DHA, DPA or EPA source and a choline (salt or ester) for use in the treatment of neurotrauma, traumatic brain injury cerebral palsy or spinal cord injury. A composition comprising a butyrate producing dietary fibre is not disclosed.

There is a need for specific products which contribute to improve, maintain or at least reduce a decline in the functioning of a mammal's, in particular a human's, limbs.

The inventors found that a specific combination of components has a positive effect on one or more functions of the limbs in order to improve specific aspects in relation to the functioning of brain functioning, in particular coordination, balance, grip strength, fine motor skills or gross motor skills.

Accordingly the invention relates to a combination of a uridine source selected from the group of uridine, deoxyuridine, cytidine, deoxycytidine, UMP, UDP, UTP, CMP, CDP, CTP, dUMP, dUDP, dUTP, dCMP, dCDP and dCTP, wherein optionally one or more hydroxyl moieties of the (deoxy)ribose of said uridine source are acylated with a C1-C24 carboxylic acid, an omega-3-polyunsaturated fatty acid having 18-24 carbon atoms, which omega-3 polyunsaturated fatty acid is selected from the group consisting of docosahexaenoic acid (DHA), docosapentaenoic acid (DPA) and eicosapentaenoic acid (EPA), and a butyrate producing dietary fibre
for at least on use selected from

- the prevention or treatment of a disturbance in coordination of limbs in a mammal;
- the prevention or treatment of a disturbance in equilibrium in a mammal;
- the prevention or treatment of a disturbance in limb strength, in particular forelimb grip strength in a mammal;
- the prevention or treatment of a disturbance in a fine-motor skill in a mammal; and
- the prevention or treatment of a disturbance in a gross-motor skill in a mammal.

Said combination of the uridine source, the omega-3-polyunsaturated fatty acid having 18-24 carbon atoms and the butyrate producing dietary fibre may be used in the manufacture of a medicament or a preparation for at least one of these uses.

In practice, the combination for use according to the invention, comprises administering an effective amount of the combination to the mammal. Such mammal is typically in need of treatment because it suffers from a disturbance in coordination of limbs in a mammal, a disturbance in equilibrium in a mammal, a disturbance in limb strength, in particular forelimb grip strength in a mammal, a disturbance in a fine-motor skill in a mammal, or a disturbance in a gross-motor skill, or because the mammal belongs to a risk group for developing such a disturbance. Such risk groups are in particular mammals suffering from a disorder of which any one or more of said disturbances are known symptoms. In particular, mammals suffering from a disorder mentioned in the present description or claims belong to such risk groups.

It has surprisingly been found that a combination of the omega3-PUFA, thea uridine source and the butyrate producing fibre, said combination optionally comprising one or more further active ingredients, such as vitamin D , has a positive effect on functioning of a limb. It is considered that the improvement is at least partly due to an improvement in the neurological functioning of the mammal treated with the combination.

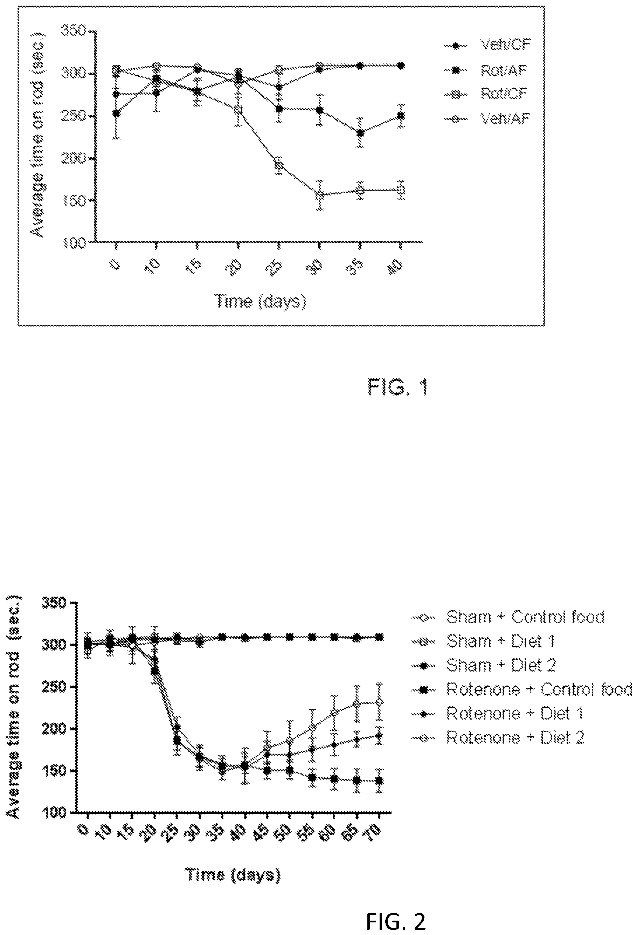

Figure 1 shows results for Example 1: the average time on rotarod (in seconds) at different times (in days) after intra-striatal injection with vehicle (Veh) or rotenone (Rot). Four groups of mice are shown as explained in Table 1. Figure 2 shows results for Example 5, regarding the time spent by mice on a Rotarod in a therapeutic setting.

Motor skills are movements and actions of the muscles. They are categorized in two groups: gross motor skills and fine motor skills. Gross motor skills involve movement of the arms, legs, feet, or entire body. This includes actions such as running, crawling, walking, swimming, and other activities that involve larger muscles. Fine motor skills are the small movements that occur in the hands, wrists, fingers, feet, toes, lips and tongue. They are the smaller actions that occur such as picking up objects between the thumb and finger, using a pencil to write carefully, holding a fork and using it to eat, and other small muscle tasks that occur on a daily basis.

A suitable way to test balancing ability in a mammal (under lab conditions), and thus also to test whether a certain treatment has an effect on a disturbance in equilibrium is a rotarod test, as described in detail in the Examples.

In humans, balance abilities are measured with the Berg Balance Scale (BBS), which is generally considered the gold standard. The BBS is a clinical measure of a person's static and dynamic balance ability. The test takes 15-20 minutes and comprises a set of 14 simple balance related tasks, ranging from standing up from a sitting position, to standing on one foot. The degree of success in achieving each task is given a score of zero (unable) to four (independent), and the final measure is the sum of all of the scores" (http://aahf.info/pdf/Berg Balance Scale.pdf) The BBS is a validated measure to assess balance abilities in neurological disorders such as Parkinson's disease (Qutubuddin et al., Arch Phys Med Rehabil., 2005, Apr; 85(4):789-92 Other tests include the Tinetti Mobility test, which assess also risk of falls in Parkinson disease (Kegelmeyer et al., 2007, Phys Ther. 87(10): 1369-78

Fine motor skills can be evaluated in humans using a panel equipped with digital timing sensors, collecting information on speed and accuracy during specific dexterity tasks as described in Smith et al., Neurology, 53(7): 1458-1458, 1999.

"Coordination" as used herein generally means "coordination of limbs". Coordination of limbs can be determined by testing the patient's ability to perform rapidly alternating and point-to-point movements correctly. Point-to-point movements are movements performed with the same index finger to bring from one point to another, for instance from the subject's own nose to the examiner's outstretched finger.

Strength can be tested with a calibrated strength tester, designed for testing the strength of a specific limb or part thereof.

"Forelimb grip-strength" can be tested with a calibrated grip strength tester. In a mouse model a grip tester (Panlab, Cornella, Spain) is used by prompting the mouse to grip a trapeze bar with both forelimbs and pulling the mouse by the tail (as proximal to the body as possible) parallel to the orientation of the strain gauge and the trapeze bar. For mean grip strength analyses, a set included five repetitions and relative mean grip strength, i.e. the absolute mean grip strength divided by body weight (grams) was calculated (Leiter et al. 2011) In human, grip strength can be measured with a hand dynamometer.

The term "a" or "an" as used herein is defined as "at least one" unless specified otherwise.

When referring to a noun (*e.g.* a compound, an additive *etc.*) in the singular, the plural is meant to be included.

The term "or" as used herein is to be understood as "and/or" unless specified otherwise.

For the purpose of clarity and a concise description features are described herein as part of the same or separate embodiments, however, it will be appreciated that the scope of the invention may include embodiments having combinations of all or some of the features described.

The term 'fatty acid' is used herein in a way as is common in the prior art. Thus, the fatty acid may be provided as a free fatty acid or salt thereof or in a derivative form - suitable for use in a nutritional of pharmaceutical product - which is derivative form is degradable in the body to release the fatty acid. Suitable derivative forms include esters and ethers, including monoglyceride, diglyceride, triglyceride and phospholipid forms, as known in the art. In particular, good results have been achieved with a triglyceride. When making calculations about amounts, one can assume the same bioavailability as the pure fatty acid and a contribution of fatty acid that is similar to the amount moles of fatty acids in the complete molecule, and correcting for the weight of the complete molecule. Polyunsaturated fatty acids are abbreviated as PUFA's.

The invention is in particular considered useful for treatment of a human, preferably a human of at least 18 years of age, in particular a human of at least 50 years of age, more in particular an elderly human (at least 65 years of age).

When referred to a dosage of a component, the specified dosage is in particular useful for treatment of a human, more in particular for an adult human, unless indicated otherwise.

The combination for use according to the invention is usually administered at least about once a week. Preferably it is at administered at least once per period of three days, more preferably at least once per period of two days, in particular at least once per day, more in particular at least twice per day. In general, the combination is administered up to 10 times, preferably up to 8 times, in particular 5 times per day or less. For embodiments wherein the combination or composition is administered less than once a day, the unit dosages (dosage per serving) of the active ingredients is usually within the range for the daily dosages mentioned elsewhere herein, although the concentration of the active ingredients may be higher.

A combination for use according to any of the invention can be administered as part of a nutritional composition. The nutritional composition may be a fluid drinkable composition, a spoonable composition or a solid composition. Preferably, the nutritional composition is a liquid composition, preferably liquid ready to drink composition. The combination or nutritional composition according to the invention, is preferably administered to the subject in need thereof by oral ingestion. In an alternative embodiment, tube feeding is used.

In particular, in case a nutritional composition for use in accordance with the invention is intended for prophylactic or therapeutic treatment of symptom of a disease, the composition may be a medical food product, *i.e.* a food product for use in enhancing, maintaining or restoring health and/or prevent a disease, prescribed by a health care professional like a physician, nurse, or dietician, and destined for and supplied to persons in need thereof.

In yet another embodiment, the combination is administered rectally.

In a preferred embodiment, directed to the combination for a medical use, the mammal is a human suffering from a neurological disorder or a psychiatric disorder or a human at risk of suffering from such disorder, benefits from treatment with a combination for use according to the invention. As used herein 'at risk' means in particular a significantly above average risk.

The neurological disorder is preferably a peripheral neuropathy, an autonomic neuropathy, or an enteric nervous system neuropathy, preferably an autonomic neuropathy, or an enteric nervous system neuropathy.

Preferably, the neurological disorder is a progressive neurodegenerative disease.

Preferably, the neurological disorder is selected to from the group consisting of synucleopathies, diabetic neuropathy, Duchenne's dystrophy, cerebrovascular disease (more particularly stroke), Multiple Sclerosis, pure autonomic failure, and spinal cord injury, preferably selected to from the group consisting of cerebrovascular disease (more particularly stroke), Multiple Sclerosis, pure autonomic failure, spinal cord injury, and Parkinson's disease.

In particular, good results have been achieved with a combination of the invention using a mouse model for Parkinson's disease. In a particularly preferred embodiment, the combination of the invention is useful to improve coordination or balance in a Parkinson's patient.

In a specific embodiment, the combination is for use of a subject suffering from cerebral palsy.

Preferably, the psychiatric disorder is a pervasive development disorder, preferably an austistic spectrum disorder.

Preferably, the psychiatric disorder is depression, in particular a depressive mood disorder.

In a further preferred embodiment, the combination of the invention is for use in the treatment of a human suffering from dementia, in particular Alzheimer Disease. Based on tests with Alzheimer patients, the inventors consider that dementia patients, in particular may benefit from treatment with a combination of the invention with respect to a fine motor skill or limb strength, such as handgrip strength.

The PUFA, in particular DHA, EPA and/or DPA, is preferably provided as triglyceride, diglyceride, monoglyceride, free fatty acid or salt or ester, phospholipid, lysophospholipid, glycerol ether, lipoprotein, ceramide, glycolipid or combinations thereof.

More preferably, the combination for use according to the invention comprises at least the omega3-PUFA, in particular DHA, in triglyceride form.

The present combination for use according to the invention preferably comprises 1-40 wt.% DHA based on total fatty acids, preferably 3-36 wt.% DHA based on total fatty acids, more preferably 10-30 wt.% DHA based on total fatty acids.

The present combination for use according to the invention preferably comprises 0.5-20 wt.% EPA based on total fatty acids, preferably 2-10 wt.% EPA based on total fatty acids, more preferably 5-10wt.% EPA based on total fatty acids.

The proportion of DHA+EPA of the total fatty acids present in the combination for use according to the invention usually is 55 wt. % or less, preferably 5-50 wt.%, more preferably 10-45 wt.%, most preferably 15-40 wt.%.

The daily dosage of polyunsaturated fatty acid having 18-24 carbon atoms administered via the combination usually is in the range of 0.4-15 grams, preferably in the range of 0.6-12 g, more preferably in the range of 1.0-10 g, in particular in the range of 1.5-5 g.

In particular, good results have been achieved with DHA, especially in combination with butyrate producing fibre. If present in the combination for use according to the invention DHA is preferably present in sufficient amount for the administration of DHA in an amount of 300 to 4000 mg per day, more preferably 500-2500 mg per day.

If EPA and/or DHA are present, the total daily dosage of DHA plus EPA taken together generally is in in the range of 300-7500 mg/day, preferably in the range of 500-6500 mg/day, more preferably in the range of 1000-5000 mg/day, in particular 1000 - 4000 mg/day, more in particular 1500-3000 mg/day.

In particular, good results have been achieved with a combination comprising both EPA and DHA. If both are present, the weight to weight ratio EPA:DHA usually is in the range of 1:99 to 99:1, preferably in the range of 1:10 - 5:1, in particular in the range of about 1:5 - 1:2, more in particular about 1:3 - 1:2.

In terms of DHA content in a nutritional composition that is administered in accordance with the invention, the DHA content preferably is 5 g/100 gram nutritional composition or less, in particular 1-4 g/100 g nutritional composition.

Preferably, a combination for use according to the invention comprises fish oil providing the omega-3-PUFA. Another particularly suitable source for the omega-3-PUFA is algae oil.

The present combination for use according to the invention preferably comprises less than 5 wt.% arachidonic acid based on total fatty acids, more preferably below 2.5 wt.%, e.g. down to 0.5 wt%.

Preferably the weight ratio DHA/AA in the present combination for use according to the invention is at least 5, preferably at least 10, more preferably at least 15, preferably up to e.g. 30 or even up to 60. Evidently, the ratio may be higher and approximate infinity if AA is absent (non-detectible).

The ratio omega-6/omega-3 fatty acids in the present combination for use according to the invention is preferably below 0.5, more preferably below 0.2, e.g. down to 0.05 or to 0.01. In particular, the ratio omega-6/omega-3 fatty acids (C 20 and higher) in the present combination for use according to the invention is preferably below 0.3, more preferably below 0.15, e.g. down to 0.06 or to 0.03.

The uridine source is selected from (deoxy)uridine, nucleotides of (deoxy)uridine, cytosine, (deoxy)cytidine, nucleotides of (deoxy)cytidine and derivatives thereof, which derivatives are acylated (esterified), wherein one or more hydroxyl moieties of the (deoxy)ribose of said uridine source are acylated with a C1-C24 carboxylic acid. Dietary cytidine, which is capable of being converted into uridine in humans, is regarded as a source of uridine in the context of the present invention. Preferred acylated forms of the uridine source are those wherein the (deoxy)ribose has been acylated with acetic acid, n-caproic acid, caprylic acid, or n-capric acid, because these increase the bioavailability of the uridine source. Methods for reacting these medium chain fatty acids to uridines, for example to the 5' position of the uridine are known in the art per se for other fatty acids and comprise conventional acylation methods. In a further embodiment the uridine source is acylated with a PUFA, for instance an omega-3 PUFA. Thus, in a specific embodiment, the combination for use of the invention comprises the PUFA and the uridine source combined in a single molecule, which may be hydrolysed *in vivo.* Esters of a uridine/cytidine derivatives and methods for making those are generally known in the art. Such derivatives have amongst others been described in EP 1,390,378 and in US 5,470,838.

Preferably, theuridine source is selected from the group of uridine, UMP, UDP, UTP, CMP, CDP, CTP dUMP, dUDP, dUTP, dCMP, dCDP and dCTP, wherein optionally one or more hydroxyl moieties of the (deoxy)ribose of said nucleotide or nucleoside is acylated.

Particularly preferred is a uridine source selected from the group of uridine UMP, UDP, UTP, dUMP, dUDP, dUTP, wherein optionally the (deoxy)ribose of said nucleotide or nucleoside is acylated.

In a highly preferred embodiment, the present combination for use according to the invention comprises uridine and/or uridine phosphate.

Preferably at least 50 wt.% of the uridine source in the combination for use according to the invention is provided by UMP, more preferably at least 75 wt.%, most preferably at least 95 wt.%.

The present combination for use according to the invention is preferably administered (or is in a format) to provide uridine (the cumulative amount of uridine, deoxyuridine, uridine phosphates, uracil and acylated uridine derivatives) in an amount of 0.08-3 g per day, preferably 0.1-2 g per day, more preferably 0.2-1 g per day.

The present combination for use according to the invention preferably comprises uridine in an amount of 0.08-3 g UMP per 100 ml of a liquid product, preferably 0.1-2 g UMP per 100 ml of a liquid product, more preferably 0.2-1 g per 100 ml liquid product.

Preferably 1-37.5 mg UMP per kilogram body weight is administered per day.

The required dosages of the equivalents on a weight base can be calculated from the dose for UMP by taking equimolar amounts using the molecular weight of the equivalent and of UMP, the latter being (about) 324 Dalton. Accordingly a daily dosage of the uridine source in the range of 3-116 $\mu$mol per kg body weight per day is preferred. In a particularly preferred embodiment the daily dosage of uridine source is 10-50 $\mu$mol per kg body weight per day . If desired a lower amount or higher amount, e.g. from about 1 to about 150 $\mu$mol/kg body weight can be administered.

As follows from the above, the combination for use according to the invention comprises dietary fibre.

The fibres may be selected from soluble fibres and insoluble fibres.

The fibres are generally composed of a plurality of carbohydrate units. The fibres may be short-chain indigestible carbohydrates or long-chain indigestible carbohydrates. Dependent on the type of fibre, national food regulations may have different definitions for what constitute short chain or long chain. As used herein, short chain fibres generally have a polymerization degree of less than 20, in particular of 2-12 less, more in particular 3-9; long chain fibres have a polymerization degree at least one higher than specified for short chain fibres, so 10 or more, 13 or more or 20 or more.

The inventors surprisingly found that the presence of a specific group of fibres in combination with the omega-3-PUFA and the uridine source has a positive effect on specific body functions wherein the limbs play a role. As illustrated by the Examples, mice treated with a diet containing butyrate producing fibre performed better in a rotarod test than mice treated with a diet without such fibre, indicating improved coordination and/or body balance (equilibrium). From the Examples, the inventors realise that in particular Parkinson patients may benefit from the butyrate producing fibre in combination with the uridine source, omega3-PUFA and optionally one or more other components, such as vitamin D. Without being bound by theory, the inventors contemplate that these fibres produce butyrate which is absorbed by the body and provides a neuroprotective effect relevant to improve coordination or body balance. The inventors further consider that butyrate producing fibre is positive for improving for limp grip strength or a fine motor skill.

Butyrate producing fibres are indigestible carbohydrates that have butyrate as a breakdown product when fermented by colonic flora. The term 'butyrate producing fibre' is used herein in particular for fibre that is capable of producing at least 0.5 mmol or more, preferably at least 0.75 mmol or more, more preferably at least 1.0 mmol or more butyrate / gram fibre after 24 hours of *in vitro* fermentation. In practice, the maximum amount of butyrate producible under these conditions is usually 5 mmol or less, in particular 3.5 mmol or less, more in particular 2.5 mmol or less butyrate / gram fibre after 24 hours of *in vitro* fermentation.

The producible amount of butyrate is determinable using a semi-dynamic colon model (e.g. using a TIM artificial gut system). In an alternative model, fresh faecal samples are collected from healthy adults (without gastrointestinal problems; no use of antibiotics for a last 2 weeks prior to sample taking). Faecal samples are divided in smaller portions and mixed with glycerol (10%) in an anaerobic cabinet and stored at -80°C. In each experiment the faecal samples from all donors are pooled at equal concentrations and mixed together in an anaerobic cabinet, to avoid subject-dependent variation in the adult microbiota as much as possible. The samples are inoculated with a fibre under fermentation conditions and fermentation is allowed to take place. Butyrate production is determined after 24 hrs using GC. In particular

- the model makes use of samples from four healthy adults (e.g., three male donors and one female donor) in the age of 19-35 years;
- the fibre is inoculated in a faeces suspension at a content of 200 mg/ 6 ml faeces suspension
- the faeces suspension is made by mixing aeces with a fermentation medium as 1:5 v/v;
- the fermentation medium: buffered peptone water 3.0 g/l, Yeast Extract 2.5 g/l, Tryptone 3.0 g/l, L-Cysteine-HCl 0.4 g/l, Bile salts 0.05 g/l, $K_2HPO_4.3H_2O$ 2.6 g/l, $NaHCO_3$ 0.2 g/l, NaCl4.5 g/l, $MgSO_4.7H_2O$ 0.5 g/l, CaCl2. $2H_2O$ 0.3 g/l, $FeSO_4.7H_2O$ 0.005 g/l. Ingredients can be added one by one in 800 ml water, pH is adjusted to 6.3±0.1 with $K_2HPO_4$ or $NaHCO_3$ and volume is filled up to 1 liter. Medium is sterilized for 15 minutes at 121°C
- The fermentation temperature is 37°C

The total dosage of butyrate producing fibre in accordance with the invention preferably is 1 to 15 g per dosage, more preferably of 2 to 10 g per dosage, in particular 3-8 g per dosage. The total butyrate producing fibre content, based on total fibre content in a composition for use in accordance with the invention is up to 100 wt %, in particular 99 wt. % or less, more in particular 95 wt. % or less. The total butyrate producing fibre content, based on total fibre content preferably is at least 50 wt. %, based on total fibre content, preferably at least 70 wt. %, more preferably at least 80 wt. %, in particular 90 wt. % or more.

Preferably the combination or nutritional composition for use according to the invention comprises fibre in an amount sufficient to produce 0.3-5 mmol, more preferably 0.5-3.5, in particular 0.7-3, more in particular 1.0-2.5 mmol butyrate per gram fibre, under the using a semi-dynamic colon model, described above.

Preferred soluble butyrate producing fibres include fructooligosaccharides (FOS), galactooligosaccharides (GOS), bran and dextrins (*e.g.* Nutriose®). These are well soluble and are a suitable substrate for colonic flora to produce butyrate, when fermented. Preferred brans are oat bran, rice bran and wheat bran. In a particularly preferred embodiment, several butyrate producing fibres are used, such as at FOS and a dextrin plus optionally bran and/or GOS; or FOS and bran plus optionally dextrin and/or GOS. Good results have been achieved with a combination of short chain FOS, long chain FOS, oat bran, GOS and dextrin. In an other preferred embodiment, the combination is part of a nutritional composition that comprises one or more butyrate producing fibres but that is essentially free of GOS. Such composition preferably is a milk-free composition.

Preferred insoluble butyrate producing fibres include resistant starch, such as high-amylose starch or retrograded or RS3 starch. Resistant starch is suitable to provide a particularly high butyrate production per g of resistant starch.

Resistant starch is defined to be as those starches which remain intact after digestion during 2 hours in the system of Englyst et al Am J Clin Nutr 1999, 69, 448-454. Commercially available resistant starches are Actistar and Novelose 330.

Preferred resistant starches are resistant starches from rice or corn.

In a specific embodiment, the resistant starch comprises more than 50wt% linear polymers of alpha 1,4 glucans which have a degree of polymerization between 10 and 35. Suitable sources of such resistant starches are beans, peas, heat-treated potatoes and heat-treated cereals. Simultaneous presence in the colon of resistant starch and beta glucans, in combination with a xylan will support of growth of the right type of butyrate generating bacteria species.

.Preferably a combination of the invention, in particular a nutritional composition to be used according to the invention further comprises a vitamin, preferably at least one vitamin selected from the group of, vitamin E, vitamin C, vitamin D, vitamin B12, vitamin B6 and folic acid.

Advantageously, vitamin B6, vitamin B12 and folate are included. It has been found in particular that a nutritional composition comprising these three vitamins has a beneficial effect on fine motor skills or in improving strength, such as handgrip. In particular, Alzheimer Disease patients benefit from the presence of these vitamins in a combination of the invention, although their presence may also benefit other subjects, in particular other subjects suffering from a neurological or psychiatric disease with respect to a fine motor skill, strength or other use of the invention.

A combination for use in accordance with the invention preferably comprises 50 to 1000 pg folic acid, more preferably 150 to 750 pg, most preferably 200 to 500 pg folic acid, per 100 ml liquid product.

The daily folic acid dosage in accordance with the invention preferably is 50-1000 pg folic acid per day, more preferably 150-750 pg, most preferably 200 - 500 pg folic acid per day.

The present nutritional composition preferably comprises 0.5 to 15 pg vitamin B12, more preferably 1 to 10 pg, most preferably 1.5 to 5 $\mu$g vitamin B12, per 100 ml liquid product.

The daily vitamin B12 dosage in accordance with the invention preferably is 0.5-15 pg vitamin B12 per day, more preferably 1-10 pg, most preferably 1.5-5 pg vitamin B12 per day.

Advantageously, the nutritional composition in a use according to the invention comprises 0.5 to 3 mg, preferably 0.5-2 mg vitamin B6, per 100 ml liquid product.

Preferably, each of vitamin B6, vitamin B12 and folate are included.

In a highly preferred embodiment, the combination for use in accordance with the invention comprises vitamin D. An advantage of vitamin D is a reduced balance impairment and reduced risks of falls. In particular, the inventors realized that Parkinson patients benefit from vitamin D with respect to these effects, although other subjects - in particular humans suffering from another neurological disorder or a psychiatric disorders - may also have a benefit from vitamin D with respect to a use of the invention.

Further, the inventors found a positive effect of vitamin D in a combination of the invention on grip strength. In particular, the presence of vitamin D in a combination of the invention is considered beneficial to limb strength of an elderly person, a Parkinson patient or a dementia patient, e.g. an Alzheimer patient. It is in particular surprising the vitamin D has an effect in an elderly subject, a dementia patient or a Parkinson patient. Earlier scientific papers reported that vitamin D did not have an effect on strength in elderly people (Smedshaug et al, Food & Nutrition, VOl 51, No 2 (2007) p 74-78 ...

Good results have been obtained with vitamin D3 (cholecalciferol, calcifediol, calcitriol).

If present, the concentration of vitamin D, preferably of vitamin D3, in a nutritional composition for use in accordance with the invention usually is in the range of 5-110 $\mu$g/100, in particular in the range of 6-85 $\mu$g/100g preferably in the range of 10-50$\mu$g/100g, more preferably 15-45 $\mu$g/100g.

The combination for use according to the invention usually provides vitamin D to the mammal, in particular human, that is treated in a in a daily dosage of up to about 50 $\mu$g, preferably 25 -40$\mu$g. For a liquid product, the vitamin D3 content preferably is 5-85 $\mu$g per unit dosage. The unit dosage of a liquid product preferably has a volume of 50-250 ml, in particular 100-150 ml.

In a specific embodiment, the nutritional composition for use according to the invention comprises one or more trace elements, in particular selenium.

Preferably, the nutritional composition comprises at least 2, 3, 4, 5, or 6 components selected from the group o of phospholipids, vitamin E, vitamin C, selenium, vitamin B12, vitamin B6 and folic acid, even more preferably comprises phospholipids, vitamin E, vitamin C, selenium, vitamin B12, vitamin B6 and folic acid.

In a specific embodiment, the nutritional composition contains choline. Preferably the present nutritional composition contains choline and/or phosphatidylcholine. Preferably the nutritional composition is administered (or is in a format) for providing more than 50 mg choline per day, preferably 80-2000 mg choline per day, more preferably 120-1000 mg choline per day, most preferably 150-600 mg choline per day.

Preferably the present nutritional composition contains 50 mg to 3 gram choline per 100 ml of a liquid formula, preferably 200 mg - 1000 mg choline/100 ml.

The present nutritional composition may advantageously contain phospholipids. As used herein, the term phospholipid includes lyso-phospholipids, de-acylated phospholipids and glycerophospholipids. Preferably a phospholipid is provided in the form of lecithin. The present nutritional composition is preferably a liquid composition, wherein lecithin is provided in an amount of 0.01 and 1 gram lecithin per 100 ml, more preferably between 0.05 and 0.5 gram lecithin per 100 ml. The presence of lecithin is in particular preferred in case a human suffering from progressive neurodegenerative disease, such as Parkinson's disease is treated.

Further, preferred phospho¬lipids are selected from the group of phosphatidylcholine(PC), phosphatidylethanolamine (PE), phosphatidylserine (PS), phosphatidic acid (phosphatidate), phosphoinositides (such as phosphatidylinositol (PI), phosphatidylinositol phosphate, phosphatidyl¬inositol bisphosphate, phosphatidylinositol triphosphate) and sphingomyelin. In particular an elderly subject or a dementia patient, such as an Alzheimer patient may benefit from any one or more, preferably two or more of the these phospholipids.

The nutritional composition usually further comprises a digestible carbohydrate fraction (e.g. sugars, digestible starch, which may be partially hydrolysed) and/or a proteinaceous matter fraction (peptides, proteins, amino acids, including derivatives thereof that still contribute to energetic value of the composition, e.g. hydrolysable esters). Further, the composition may comprise a lipid fraction comprising the PUFA and one or more other lipids.

In an advantageous embodiment, the lipids account for less than 50 en% of the nutritional composition, in particular 46 wt. % or less, more in particular 43 wt. % or less, or 40 wt. % or less. Usually, the lipids (including he omega-3 PUFA) provide at least 1 en %, preferably at least 5 en %, in particular at least 10 en % of the nutritional composition. Proteinaceous matter and/or digestible carbohydrates (non-lipid component having a caloric value) typically provide the balance of the energetic value. For energetic value the Atwater factors are used: for digestible carbohydrates (4 kcal/g), proteins (4 kcal/g) and lipids (9 kcal/g), and zero for others, such as the organic acids and nutritional fiber, minerals and vitamins.

In a specific embodiment, the nutritional composition for use in accordance with the invention comprises

   i-) at least one component selected from the group of uridine, acylated uridine, uridine monophosphate and acylated uridine monophosphate.
   ii) DHA and EPA
   iii) a vitamin B selected from the group of vitamin B6, vitamin B9 and vitamin B12
   iv) a phospholipid
   v) an antioxidant selected from the group of vitamin C, vitamin E and selenium
   vi) a choline
   vii) a protein.

Such nutritional composition is particularly useful for treatment of an Alzheimer patient in accordance with the invention, also if such product is essentially free of butyrate producing fibres and/of vitamin D.

The composition of this specific embodiment preferably comprises phosphatidylcholine, and the weight to weight ratio phosphatidylcholine to choline preferablt is more than 0.26.

The total content of uridine source in this specific embodiment preferably is in the range of 5-30 mg per gram dry weight, more preferably in the range of 8-20 mg per gram dry weight, in particular in the range of 10-18 mg per gram dry weight.

The nutritional composition of this specific embodiment preferably comprises 5-10 mg EPA per gram dry weight and 2.5-20 mg DHA per gram dry weight.

The nutritional composition of this specific embodiment preferably has a protein content is less than 400 mg per gram dry matter, preferably 100-340 mg protein per gram dry matter. Preferably whey protein is present. Further advantageous proteins in particular include fish proteins (in particular cod protein), egg-protein and vegetable proteins. The total content of non-dairy protein preferably is more than 21 wt. % ,more preferably 22-80 wt. %, in particular 25-40 wt. %.

The nutritional composition of this specific embodiment preferably comprises 0.15-0.5 g digestible carbohydrate per gram dry weight, in particular 0.20-0.40 g digestible carbohydrate per gram dry weight.

The nutritional composition of this specific embodiment preferably comprises 0.15-0.3 g lipids per gram dry weight.

The energy density of a composition according to this specific embodiment typically is less than 13 kcal per gram dry matter, preferably of 3-9.3 kcal per gram dry matter, more preferably of 4.0-7.0 kcal per gram dry matter.

For ease of administration to a patient, in particular an Alzheimer patient, the nutritional composition of this specific embodiment is preferably administered as a fluid. It may be packaged as a ready-to-use fluid or be an instant product (powder) to be reconstituted prior to use. The fluid typically has a dry matter content of 15-30 g per 100 ml, in particular of 16-24 g per 100 ml, more in particular of 17-22 g per 100 ml. The osmolarity of the fluid composition typically is 120 to 450 mEq/l.

The invention is now illustrated by the following examples.

Example 1

In mice, Parkinson's disease (PD), was induced by intra-striatal injection of 2.7 $\mu$g of rotenone by stereotaxic surgery into the brain. Epidemiological studies had previously shown that exposure to this pesticide induced Parkinson's disease (PD). The rotarod treadmill consists of a plastic rod, with a non-slippery surface, above the base (trip plate). Mice were placed on an accelerating rod with speeds starting with 2 rpm and gradually increasing to 20 rpm. The rodent's ability to remain on the rotating rod (time to first fall) was recorded for a maximum of 5 minutes. Before baseline, mice were trained with the rotarod apparatus to rule out learning effects throughout the experiment.

Four experimental groups of 10 mice each were used which were either injected with rotenone or vehicle and which received control or active diet (=UMP+ fish oil) according to Table 1. The specific composition of the control and active diets are presented in Table 2. Dietary intervention was started one week before surgery and was continued for six weeks.

Table 1 Experimental groups

| Group | Intra-striatal injection | Diet |
|---|---|---|
| Veh/CF | Vehicle | Control |
| Veh/AF | Vehicle | UMP + fish oil |
| Rot/CF | Rotenone | Control |
| Rot/AF | Rotenone | UMP + fish oil |

| Table 2 Composition of the diets | | |
|---|---|---|
| | | |
| | | |
| animal food based on AIN -93 M Reeves, et al. (1993) | **g/100g diet** | **g/100g diet** |
| | **Control** | **Fish+UMP** |
| Corn starch | 31.10 | 29.30 |
| Casein (>85% protein) | 14.00 | 14.00 |
| corn dextrine | 15.50 | 15.50 |
| Sucrose | 10.00 | 10.00 |
| Dextrose | 10.00 | 10.00 |
| | | |
| Fibers | 5.00 | 5.00 |
| | | |
| Minerals mix (AIN-93M-MX) (*) | 3.50 | 3.50 |
| Vitamins mix (AIN-93-VX) (*) | 1.00 | 1.00 |
| | | |
| **Fat** | 4.77 | 4.77 |
| saturated fat | 1.3 | 1.2 |
| Monounsaturated fatty acids (MUFA) | 1.100 | 1.100 |
| PUFA | 2.300 | 2.200 |
| EPA (C-20:5w3) | 0.000 | 0.300 |
| DHA (C-22:6w3) | 0.000 | 0.700 |
| | | |

(continued)

| Table 2 Composition of the diets | | |
|---|---|---|
| | | |
| **Additions:** | | |
| L-cystine | 0.180 | 0.180 |
| Choline bitartrate (41.1% choline) | 0.250 | 0.250 |
| Tert-butylhydroquinone | 0.0008 | 0.0008 |
| | | |
| | | |
| UMP disodium (24%H2O) | 0 | 1.0 |
| | | |
| Total | 100.0 | 100.0 |
| Energy | 344.6 | 345.5 |
| (*) See Reeves 1993 - J Nutr; Nov; 123(11); 1939-51. | | |

The intra-striatal injection with rotenone resulted in the well-known motor symptoms of PD, shown by the impaired ability of the mice to remain on a moving rotarod. The ability to remain on the rod was much better (more than 50 % higher average time on the rod) in mice treated with a combination of the invention (Ror/AF) after treatment, as illustrated by Figure 1.

Example 2:

Ready to use liquid product providing per 100 mL:

Energy: 50-120 kcal
Protein: 0-10 g
Lipids: 1-5 g
Digestible carbohydrates: 4-30 g
and which comprises:

$$\mathrm{DHA+EPA+DPA= 1000\text{-}2000 \ mg}$$

Uridine monophosphate (disodium salt) or uridine 0.5 g

Example 3

A preferred nutritional composition according to the invention comprises, per daily dose or per 100 ml composition:

100 - 500 mg, preferably 200-400 mg EPA,
900 - 1500 mg, preferably 950-1300 mg DHA
50 - 600 mg, preferably 60-200 mg phospholipids,
200 - 600 mg, preferably 300-500 mg choline,
400 - 800 mg, preferably 500-700 mg UMP or an equivalent molar amount of uridine,
20 - 60 mg, preferably 30-50 mg vitamin E (alpha-TE),
60 - 100 mg, preferably 60-90 mg vitamin C,
40 - 80 pg, preferably 45-65 pg selenium,
1-5 $\mu$g, preferably 2-4 $\mu$g vitamin B12,
0.5 - 3 mg, preferably 0.5-2 mg vitamin B6, and
200 - 600 pg, preferably 300-500 pg folic acid.

Example 4

Liquid ready to use product as in Example 2 or 3 for diabetic patient suffering from neuropathy, in which the carbohydrate fraction is composed of (per 100 mL):

1.5 g galactose
3 g palatinose
1 g slowly digestible starch
0.2 g fructose
3 g maltodextrins
2 g glucose
1.05 g isomalto oligosaccharides

Example 5

The effects of a diet comprising a pyrimidine derivative (UMP) and a PUFA source (DHA) in a therapeutic setting were studied, wherein treatment with a diet according to the invention was initiated 4 weeks after injection with 5.4 μg rotenone or a vehicle and continued for seven weeks. Six experimental groups of male C57Bl/6J mice were used which were either treated with vehicle (Sham) or rotenone and which either received a control diet or a diet according to the invention (diet 1 or diet 2), as indicated in the following table:

Table 3:. Experimental groups

| Group | Intra-striatal injection | diet |
|---|---|---|
| Sham+Control Food (control) | Sham | Control |
| Sham+Diet 1 (control) | Sham | Uridine source+ DHA |
| Sham+Diet 2 (control) | Sham | Uridine source + DHA + butyrate producing fibres |
| Rotenone+Control Food | Rotenone | Control |
| Rotenone+Diet 1 | Rotenone | Uridine source + DHA |
| Rotenone+Diet 2) | Rotenone | Uridine source + DHA + butyrate producing fibres |

The compositions of the control and active diets are presented in the following Table.

| Table 4: Composition of the diets | | | |
|---|---|---|---|
| animal food based on AIN -93 M Reeves, et al. (1993) | g / 100 g diet | g / 100 g diet | g/100 g diet |
| | Control | Diet 1: Fish oil+UMP | Diet 2: Fish oil+Uridine+buty-rate producing fibers |
| Corn starch | 31.10 | 29.30 | 31.97 |
| Casein (>85% protein) | 14.00 | 14.00 | 14.00 |
| corn dextrine | 15.50 | 15.50 | 14.20 |
| Sucrose | 10.00 | 10.00 | 10.00 |
| Dextrose | 10.00 | 10.00 | 10.00 |
| | | | |
| Fibers (cellulose) | 5.00 | | |
| | | 5.00 | 0 |
| Butyrate producing fibers | 0 | 0 | 7.27[1] |
| Minerals mix (AIN-93M-MX) (*) | 3.50 | 3.50 | 3.50 |
| Vitamins mix (AIN-93-VX) (*) | 1.00 | 1.00 | 1.00 |

(continued)

| Table 4: Composition of the diets | | | |
|---|---|---|---|
| animal food based on AIN -93 M Reeves, et al. (1993) | g / 100 g diet | g / 100 g diet | g/100 g diet |
| | | | |
| Fat | 4.77 | 4.77 | 4.77 |
| saturated fat | 1.3 | 1.2 | 1.2 |
| Monounsaturated fatty acids (MUFA) | 1.1 | 1.1 | 1.1 |
| PUFA | 2.3 | 2.2 | 2.2 |
| EPA (C-20:5w3) | 0.0 | 0.3 | 0.5 |
| DHA (C-22:6w3) | 0.0 | 0.7 | 0.7 |
| | | | |
| | | | |
| Additions: | | | |
| L-cystine | 0.180 | 0.180 | 0.180 |
| Choline bitartrate (41.1% choline) | 0.250 | 0.250 | 0.922 |
| Tert-butylhydroquinone | 0.0008 | 0.0008 | 0.0008 |
| Additional vitamins[2] | | | 0.688 |
| Soy lecithin | | | 0.755 |
| UMP disodium (24%H2O) | 0 | 1.0 | |
| Uridine (pure) | | | 0.511 |
| Total | 100.0 | 100.0 | 100.0 |
| Energy | 344.6 | 345.5 | 358.0 |
| [1] Butyrate producing fibres (5 gram per 100 g diet):<br>1.50 g GOS<br>0.17 g long-chain FOS<br>1.67 g short- chain FOS<br>1.67 g nutriose<br>And further 0.5 g lactose and 0.5 g the carbohydrate and moisture to 7.27 g<br>[2] Additional vitamins (per 100 g diet) | | | |

| | |
|---|---|
| Ascorbic acid (100% pure) | 0.16 g |
| Vit. E (Tocopherol acetate, 50 %) | 0.465 g |
| Vitamine D (cholecalciferol, 500.000 IU/g) | 5.6 $\mu$g |
| Vit. B6 (Pyridoxin hydrochloride, 82 %) | 0.00407 g |
| Folic acid (100%) | 0.00060 g |
| Vit. B12 (Cyanocobalamin 0,1 %) | 0.0575 g |
| Na selenite • 5 H2O | 0.00034 g |

Diet 1 was composed of the control diet plus additionally UMP and DHA (fish oil).

Diet 2 was as Diet 1, without cellulose , but instead additionally a mixture of galactooligosaccharides and fructooligosaccharides (both long chain and short chain fructooligosaccharides and instead of UMP uridine.

A rotarod test was performed for 10 weeks, starting 1 week after intra-striatal injection with rotenone or sham. As shown in Figure 2, rotenone induced motor problems, but after start of treatment with Diet 1 or Diet 2, an improvement in motoric functioning was shown (increased average time on the rod, compared to the 'Rotenone + Control Food' group).

The additional presence of the dietary fibre had an positive effect on motoric functioning.

**Claims**

1. A combination of
a uridine source selected from the group of uridine, deoxyuridine, cytidine, deoxycytidine, UMP, UDP, UTP, CMP, CDP, CTP, dUMP, dUDP, dUTP, dCMP, dCDP and dCTP, wherein optionally one or more hydroxyl moieties of the (deoxy)ribose of said uridine source are acylated with a C1-C24 carboxylic acid,
an omega-3-polyunsaturated fatty acid having 18-24 carbon atoms, which omega-3 polyunsaturated fatty acid is selected from the group consisting of docosahexaenoic acid (DHA), docosapentaenoic acid (DPA) and eicosapentaenoic acid (EPA), and
a butyrate producing dietary fibre
for a use selected from the group consisting of:

   - the prevention or treatment of a disturbance in coordination of limbs in a mammal;
   - the prevention or treatment of a disturbance in equilibrium in a mammal;
   - the prevention or treatment of a disturbance in limb strength, in particular forelimb grip strength in a mammal;
   - the prevention or treatment of a disturbance in a fine-motor skill in a mammal; and
   - the prevention or treatment of a disturbance in a gross-motor skill in a mammal.

2. A combination for use according to claim 1, wherein the mammal suffers from a neurological disorder or a psychiatric disorder.

3. A combination for use according to claim 2, wherein the neurological disorder is selected to from the group consisting of synucleopathies, diabetic neuropathy, Duchenne's dystrophy, cerebrovascular disease, Multiple Sclerosis, pure autonomic failure, Parkinson's disease, stroke, dementia and spinal cord injury.

4. A combination for use according to claim 3, wherein the psychiatric disorder is pervasive development disorder, preferably autistic spectrum disorder, or the psychiatric disorder is depression, in particular a depressive mood disorder.

5. A combination for use according to any of claims 1 to 4, wherein the omega-3 polyunsaturated fatty acid is DHA.

6. A combination for use according to any of the preceding claims, wherein the daily dosage of DHA plus EPA administered via the combination is in the range of 400 mg to 7500 mg, preferably in the range of 500 mg to 5000 mg, in particular in the range of 1000 mg to 3000 mg.

7. A combination for use according to any of the preceding claims, wherein the uridine source is selected from the group consisting of non-acylated UMP, acylated UMP's, non-acylated uridine, acylated uridine, non-acylated deoxyuridine and acylated deoxyuridine.

8. A combination for use according to any of the preceding claims, wherein the daily dosage of the uridine source is in the range of 1-150 $\mu$mol per kg body weight per day, preferably in the range of 3-116 $\mu$mol per kg body weight per day.

9. A combination for use according to any of the preceding claims, wherein the combination comprises a dietary fibre selected from the group of butyrate producing fibres, selected from the group of fructooligosaccharides (FOS), galactooligosaccharides (GOS) and dextrins (e.g. Nutriose®).

10. A combination for use according to claim 9, wherein the butyrate producing fibre is to be administered in a dosage of 3-8 grams.

11. A combination for use according to any of the preceding claims, wherein the combination is to be administered as part of a nutritional composition, further comprising a phospholipid.

12. A combination for use according to any of the preceding claims, wherein the combination is to be administered as part of a nutritional composition, further comprising at least one component selected from the group of vitamin E, vitamin C and selenium.

13. A combination for use according to any of the preceding claims, wherein the combination comprises at least one vitamin selected from the group of vitamin B12, vitamin B6 and folic acid, preferably comprising vitamin B12, vitamin B6 and folic acid.

14. A combination for use according to claim 13, wherein the combination is part of a nutritional composition, the nutritional composition comprising

> i-) at least one component selected from the group of uridine, acylated uridine, uridine monophosphate and acylated uridine monophosphate
> ii) DHA and EPA
> iii) a vitamin B selected from the group of vitamin B6, vitamin B9 and vitamin B12
> iv) a phospholipid
> v) an antioxidant selected from the group of vitamin C, vitamin E and selenium
> vi) a choline
> vii) a protein.

15. A combination for use according to any of the preceding claims, wherein the combination further comprises vitamin D, preferably vitamin D3.

16. A combination for use according to claim 15, wherein vitamin D, preferably vitamin D3, is to be administered in a daily dosage of 5-50 μg, in particular 25-40 pg.

17. A combination for use according to any of the preceding claims, wherein the combination is part of a nutritional composition, further comprising at least non-lipid components having a caloric value selected from the group of proteinaceous matter and digestible carbohydrates, wherein the total caloric value of said non-lipid components is at least 50 en%, preferably 55 en% or more, in particular 60 en% or more.

18. A combination for use according to any of the preceding claims, wherein the mammal is a human, preferably a human of at least 18 years of age, in particular an elderly human.

19. A combination for use according to any of the preceding claims, wherein the mammal is a subject suffering from cerebral palsy.

**Patentansprüche**

1. Kombination von
einer Uridinquelle, ausgewählt aus der Gruppe von Uridin, Desoxyuridin, Cytidin, Desoxycytidin, UMP, UDP, UTP, CMP, CDP, CTP, dUMP, dUDP, dUTP, dCMP, dCDP und dCTP, wobei optional eine oder mehrere Hydroxylgruppen der (Desoxy)ribose der Uridinquelle mit einer C1-C24-Carbonsäure acyliert sind,
einer Omega-3-mehrfach-ungesättigten-Fettsäure mit 18-24 Kohlenstoffatomen, wobei die Omega-3-mehrfach-ungesättigte-Fettsäure ausgewählt ist aus der Gruppe, bestehend aus Docosahexaensäure (DHA), Docosapentaensäure (DPA) und Eicosapentaensäure (EPA), und einem Butyrat herstellenden Ballaststoff
für eine Verwendung, ausgewählt aus der Gruppe bestehend aus:

> - der Prävention oder Behandlung einer Störung in der Koordination von Gliedmaßen bei einem Säugetier;
> - der Prävention oder Behandlung einer Störung des Gleichgewichts bei einem Säugetier;
> - der Prävention oder Behandlung einer Störung der Gliedmaßen-Kraft, insbesondere der Vordergliedmaßen-Greifkraft bei einem Säugetier;
> - der Prävention oder Behandlung einer Störung in einer feinmotorischen Fähigkeit bei einem Säugetier; und
> - der Prävention oder Behandlung einer Störung einer grobmotorischen Fähigkeit bei einem Säugetier.

2. Kombination zur Verwendung nach Anspruch 1, wobei das Säugetier an einer neurologischen Störung oder einer psychiatrischen Störung leidet.

3. Kombination zur Verwendung nach Anspruch 2, wobei die neurologische Störung ausgewählt ist aus der Gruppe, bestehend aus Synukleopathien, diabetischer Neuropathie, Duchenne-Dystrophie, zerebrovaskulärer Erkrankung, Multipler Sklerose, reinem autonomen Versagen, Parkinson-Krankheit, Schlaganfall, Demenz und Rückenmarksverletzung .

4. Kombination zur Verwendung nach Anspruch 3, wobei die psychiatrische Störung eine pervasive Entwicklungsstörung, vorzugsweise eine Autismus-Spektrum-Störung, ist, oder die psychiatrische Störung eine Depression, insbesondere eine depressive Stimmungsstörung, ist.

5. Kombination zur Verwendung nach einem der Ansprüche 1 bis 4, wobei die Omega-3-mehrfach-ungesättigte-Fettsäure DHA ist.

6. Kombination zur Verwendung nach einem der vorhergehenden Ansprüche, wobei die tägliche Dosierung von DHA plus EPA, verabreicht über die Kombination, in dem Bereich von 400 mg bis 7500 mg, vorzugsweise in dem Bereich von 500 mg bis 5000 mg, insbesondere in dem Bereich von 1000 mg bis 3000 mg ist.

7. Kombination zur Verwendung nach einem der vorhergehenden Ansprüche, wobei die Uridinquelle ausgewählt ist aus der Gruppe bestehend aus nicht-acyliertem UMP, acylierten UMPs, nicht-acyliertem Uridin, acyliertem Uridin, nicht-acyliertem Desoxyuridin und acyliertem Desoxyuridin.

8. Kombination zur Verwendung nach einem der vorhergehenden Ansprüche, wobei die Tagesdosis der Uridinquelle in dem Bereich von 1-150 $\mu$mol pro kg Körpergewicht pro Tag, vorzugsweise in dem Bereich von 3-116 $\mu$mol pro kg Körpergewicht pro Tag ist.

9. Kombination zur Verwendung nach einem der vorhergehenden Ansprüche, wobei die Kombination einen Ballaststoff umfasst, ausgewählt aus der Gruppe von Butyrat herstellenden Ballaststoffen, ausgewählt aus der Gruppe von Fructooligosacchariden (FOS), Galactooligosacchariden (GOS) und Dextrinen (z. B. Nutriose®).

10. Kombination zur Verwendung nach Anspruch 9, wobei der Butyrat herstellende Ballaststoff in einer Dosierung von 3-8 Gramm zu verabreichen ist.

11. Kombination zur Verwendung nach einem der vorhergehenden Ansprüche, wobei die Kombination als Teil einer Nahrungszusammensetzung zu verabreichen ist, ferner umfassend ein Phospholipid.

12. Kombination zur Verwendung nach einem der vorhergehenden Ansprüche, wobei die Kombination als Teil einer Nahrungszusammensetzung verabreicht werden soll, ferner umfassend wenigstens eine Komponente, ausgewählt aus der Gruppe von Vitamin E, Vitamin C und Selen.

13. Kombination zur Verwendung nach einem der vorhergehenden Ansprüche, wobei die Kombination wenigstens ein Vitamin, ausgewählt aus der Gruppe von Vitamin B12, Vitamin B6 und Folsäure, umfasst, vorzugsweise umfassend Vitamin B12, Vitamin B6 und Folsäure.

14. Kombination zur Verwendung nach Anspruch 13, wobei die Kombination Teil einer Nahrungszusammensetzung ist, die Nahrungszusammensetzung umfassend

    i) wenigstens eine Komponente, ausgewählt aus der Gruppe von Uridin, acyliertem Uridin, Uridinmonophosphat und acyliertem Uridinmonophosphat
    ii) DHA und EPA
    iii) ein Vitamin B, ausgewählt aus der Gruppe von Vitamin B6, Vitamin B9 und Vitamin B12
    iv) ein Phospholipid
    v) ein Antioxidans, ausgewählt aus der Gruppe von Vitamin C, Vitamin E und Selen
    vi) ein Cholin
    vii) ein Protein.

15. Kombination zur Verwendung nach einem der vorhergehenden Ansprüche, wobei die Kombination ferner Vitamin D, vorzugsweise Vitamin D3, umfasst.

16. Kombination zur Verwendung nach Anspruch 15, wobei Vitamin D, vorzugsweise Vitamin D3, in einer Tagesdosis von 5-50 $\mu$g, insbesondere 25-40 $\mu$g, zu verabreichen ist.

**17.** Kombination zur Verwendung nach einem der vorhergehenden Ansprüche, wobei die Kombination Teil einer Nahrungszusammensetzung ist, ferner umfassend wenigstens Nicht-Lipid-Komponenten mit einem Brennwert, ausgewählt aus der Gruppe von proteinartigem Material und verdaulichen Kohlenhydraten, wobei der Gesamtbrennwert der Nicht-Lipid-Komponenten wenigstens 50 En%, vorzugsweise 55 En% oder mehr, insbesondere 60 En% oder mehr, ist.

**18.** Kombination zur Verwendung nach einem der vorhergehenden Ansprüche, wobei das Säugetier ein Mensch ist, vorzugsweise ein Mensch im Alter von wenigstens 18 Jahren, insbesondere ein älterer Mensch.

**19.** Kombination zur Verwendung nach einem der vorhergehenden Ansprüche, wobei das Säugetier ein Subjekt ist, leidend an Zerebralparese,.

**Revendications**

**1.** Combinaison
d'une source d'uridine choisie dans le groupe comprenant l'uridine, la désoxyuridine, la cytidine, la désoxycytidine, et les UMP, UDP, UTP, CMP, CDP, CTP, dUMP, dUDP, dUTP, dCMP, dCDP et dCTP, dans laquelle éventuellement un ou plusieurs fragments hydroxyle du (désoxy)ribose de ladite source d'uridine sont acylés avec un acide carboxylique en C1 à C24,
d'un acide gras oméga-3 polyinsaturé ayant 18 à 24 atomes de carbone, lequel acide gras oméga-3 polyinsaturé est choisi dans le groupe constitué par l'acide docosahexénoïque (DHA), l'acide docosapenténoïque (DPA) et l'acide éicosapenténoïque (EPA), et
d'une fibre alimentaire produisant du butyrate,
pour une utilisation choisie dans le groupe constitué par :

- la prévention ou le traitement d'une perturbation de la coordination des membres chez un mammifère ;
- la prévention ou le traitement d'une perturbation de l'équilibre chez un mammifère ;
- la prévention ou le traitement d'une perturbation de la force des membres, en particulier de la force de préhension des membres antérieurs chez un mammifère ;
- la prévention ou le traitement d'une perturbation d'une habileté motrice fine chez un mammifère ; et
- la prévention ou le traitement d'une perturbation d'une habileté motrice globale chez un mammifère.

**2.** Combinaison pour une utilisation selon la revendication 1, dans laquelle le mammifère souffre d'un trouble neurologique ou d'un trouble psychiatrique.

**3.** Combinaison pour une utilisation selon la revendication 2, dans laquelle le trouble neurologique est choisi dans le groupe constitué par les synucléopathies, une neuropathie diabétique, une dystrophie de Duchenne, une maladie cérébrovasculaire, une sclérose en plaques, une dysautonomie pure, une maladie de Parkinson, un accident vasculaire cérébral, une démence, et une lésion de la moelle épinière.

**4.** Combinaison pour une utilisation selon la revendication 3, dans laquelle le trouble psychiatrique est un trouble envahissant du développement, de préférence un trouble du spectre autistique, ou le trouble psychiatrique est une dépression, en particulier un trouble dépressif.

**5.** Combinaison pour une utilisation selon l'une quelconque des revendications 1 à 4, dans laquelle l'acide gras oméga-3 polyinsaturé est le DHA.

**6.** Combinaison pour une utilisation selon l'une quelconque des revendications précédentes, dans laquelle la posologie journalière de DHA plus EPA administrés via la combinaison est située dans la plage allant de 400 mg à 7500 mg, de préférence dans la plage allant de 500 mg à 5000 mg, en particulier dans la plage allant de 1000 mg à 3000 mg.

**7.** Combinaison pour une utilisation selon l'une quelconque des revendications précédentes, dans laquelle la source d'uridine est choisie dans le groupe constitué par l'UMP non acylé, les UMP acylés, l'uridine non acylée, l'uridine acylée, la désoxyuridine non acylée et la désoxyuridine acylée.

**8.** Combinaison pour une utilisation selon l'une quelconque des revendications précédentes, dans laquelle la posologie journalière de la source d'uridine est située dans la plage allant de 1 à 150 $\mu$mol par kg de poids corporel et par jour, de préférence dans la plage allant de 3 à 116 μmol par kg de poids corporel et par jour.

9. Combinaison pour une utilisation selon l'une quelconque des revendications précédentes, laquelle combinaison comprend une fibre alimentaire choisie dans le groupe comprenant les fibres produisant du butyrate, choisies dans le groupe comprenant les fructooligosaccharides (FOS), les galactooligosaccharides (GOS) et les dextrines (par exemple Nutriose®).

10. Combinaison pour une utilisation selon la revendication 9, dans laquelle la fibre produisant du butyrate est destinée à être administrée à une dose de 3 à 8 grammes.

11. Combinaison pour une utilisation selon l'une quelconque des revendications précédentes, laquelle combinaison est destinée à être administrée en tant que partie d'une composition nutritionnelle, comprenant en outre un phospholipide.

12. Combinaison pour une utilisation selon l'une quelconque des revendications précédentes, laquelle combinaison est destinée à être administrée en tant que partie d'une composition nutritionnelle, comprenant en outre au moins un composant choisi dans le groupe comprenant la vitamine E, la vitamine C et le sélénium.

13. Combinaison pour une utilisation selon l'une quelconque des revendications précédentes, laquelle combinaison comprend au moins une vitamine choisie dans le groupe comprenant la vitamine B12, la vitamine B6 et l'acide folique, de préférence comprend de la vitamine B12, de la vitamine B6 et de l'acide folique.

14. Combinaison pour une utilisation selon la revendication 13, laquelle combinaison fait partie d'une composition nutritionnelle, la composition nutritionnelle comprenant

   i) au moins un composant choisi dans le groupe comprenant l'uridine, l'uridine acylée, le monophosphate d'uridine et le monophosphate d'uridine acylé,
   ii) le DHA et l'EPA,
   iii) une vitamine B choisie dans le groupe comprenant la vitamine B6, la vitamine B9 et la vitamine B12,
   iv) un phospholipide,
   v) un antioxydant choisi dans le groupe comprenant la vitamine C, la vitamine E et le sélénium,
   vi) une choline,
   vii) une protéine.

15. Combinaison pour une utilisation selon l'une quelconque des revendications précédentes, laquelle combinaison comprend en outre de la vitamine D, de préférence de la vitamine D3.

16. Combinaison pour une utilisation selon la revendication 15, dans laquelle la vitamine D, de préférence la vitamine D3, est destinée à être administrée à une dose quotidienne de 5 à 50 μg, en particulier de 25 à 40 μg.

17. Combinaison pour une utilisation selon l'une quelconque des revendications précédentes, laquelle combinaison fait partie d'une composition nutritionnelle, comprenant en outre au moins des composants non lipidiques ayant une valeur calorique, choisis dans le groupe comprenant les matières protéiques et les hydrates de carbone digestibles, dans laquelle la valeur calorique totale desdits composants non lipidiques est d'au moins 50 % én., de préférence 55 % én. ou plus, en particulier 60 % én. ou plus.

18. Combinaison pour une utilisation selon l'une quelconque des revendications précédentes, dans laquelle le mammifère est un humain, de préférence un humain âgé d'au moins 18 ans, en particulier un humain âgé.

19. Combinaison pour une utilisation selon l'une quelconque des revendications précédentes, dans laquelle le mammifère est un sujet souffrant d'une infirmité motrice cérébrale.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009002165 A
- WO 2009002146 A
- WO 2012125020 A
- EP 1390378 A
- US 5470838 A

**Non-patent literature cited in the description**

- **QUTUBUDDIN et al.** *Arch Phys Med Rehabil,* April 2005, vol. 85 (4), 789-92
- **KEGELMEYER et al.** *Phys Ther,* 2007, vol. 87 (10), 1369-78
- **SMITH et al.** *Neurology,* 1999, vol. 53 (7), 1458-1458
- **ENGLYST et al.** *Am J Clin Nutr,* 1999, vol. 69, 448-454
- **SMEDSHAUG et al.** *Food & Nutrition,* 2007, vol. 51 (2), 74-78
- *Reeves 1993 - J Nutr,* November 1993, vol. 123 (11), 1939-51